# DEMANDE DE BREVET EUROPEEN

(11) **EP 1 042 999 A1**
(43) Date de publication de la demande: **11.10.2000**
(21) Numéro de dépôt: 99201058.7
(22) Date de dépôt: 08.04.1999
(51) Int. Cl.: A61F 2/16

(54) **Support de lentille intraoculaire**

(71) Demandeur: Physiol, 4031 Angleur (BE)
(72) Inventeur: Nollet de Brauwere, Marc, 1640 Rhode-Saint-Genèse (BE)
(74) Mandataire: Vandeberg, Marie-Paule L.G.

(57) **Abrégé**

Un support (1) de lentille intraoculaire souple ou IOL permettant à la fois le stockage et le pliage de telles lentilles.

Ce support, généralement monté sur un flacon, comprend une tige (8) supportant deux membres flexibles (10, 12) formant pince (10, 12).

Ces membres flexibles (10, 12) portent à leur extrémité distale, tournés l'un vers l'autre, des moyens de support et d'accrochage (14) aptes à maintenir une lentille intraoculaire souple (37).

Des moyens de manoeuvre et de verrouillage actionnés par l'opérateur agissent sur ces moyens de support et d'accrochage (14), entraînant un repli de la lentille souple (37) et son maintien dans cette forme repliée.

## Description

L'invention concerne les supports de lentille intraoculaire souple. L'invention est destinée tant au stockage de telles lentilles qu'à leur manipulation dans la phase précédant leur introduction dans le volume oculaire.

La chirurgie oculaire a fait d'énormes progrès, notamment en ce qui concerne les traitements de la cataracte, du fait notamment de la mise au point de lentilles en matériaux souples permettant de remplacer les cristallins abîmés. De telles lentilles sont décrites notamment dans US-5290892.

Les lentilles souples ont comme avantage de pouvoir être introduites dans l'oeil par une incision de dimension réduite, ce qui permet de limiter les effets invasifs de telles opérations. En effet, qu'il s'agisse de matériaux hydrophiles ou hydrophobes, les lentilles souples actuelles se prêtent sans dommage à d'importantes déformations, et elles peuvent ainsi être repliées sur elles-mêmes de façon à présenter des dimensions minimales au moment de l'insertion dans l'oeil.

Différents dispositifs ont été développés à la fois pour le stockage et pour le pliage de ces lentilles oculaires (communément désignées par l'acronyme IOL, pour "Intraocular Lens").

Paradoxalement, les avantages que ces IOL présentent pour les patients (leur limpidité, leur faible dimension, leur légèreté, leur souplesse) constituent des difficultés pour l'interventionniste, car elles se révèlent par là même difficiles à manipuler.

On connaît par US-4697697 ou 4257521 des dispositifs destinés aux stockages de IOL dans lesquels la lentille est maintenue par un support dans un récipient contenant un fluide stérile (généralement, du sérum physiologique).

Le recours à ces dispositifs ne simplifie malheureusement pas les manipulations, les lentilles, repliées dans des appareils distincts, tels que décrits par exemple dans US-4844093, US-4911158, US-5578042, US-5810833 et US-5176686 devant subir un double transfert avant d'être serrées dans une pince ou autre instrument pour l'insertion dans l'oeil.

Pour éviter une telle suite de manipulations, certains fabricants ont opté pour un stockage de IOL sous une forme préalablement repliée, ce qui entraîne des problèmes de contraintes mécaniques dans les matériaux utilisés, de péremption, etc.

Il existe par ailleurs des supports combinant le stockage et le repli de la lentille. Les supports connus de ce type ont le désavantage d'une manipulation laborieuse et délicate où le risque de fausse manoeuvre est élevé.

Le but de l'invention est de mettre à la disposition des praticiens un dispositif permettant à la fois le maintien et le pliage d'IOL dans des conditions optimales. Un autre but de l'invention est d'assurer une manipulation aisée de ces lentilles et de réduire les possibilités de fausses manoeuvres.

Un autre but est de raccourcir le temps des interventions chirurgicales.

L'objet de l'invention est un support de lentille intraoculaire pliable comprenant une tige supportant deux membres flexibles formant une pince s'étendant parallèlement à un axe longitudinal. Dans ce dispositif, les membres flexibles portent, à leur extrémité distale, tournés l'un vers l'autre, des moyens de support et d'accrochage aptes à maintenir une lentille intraoculaire souple sensiblement dans un plan (de préférence perpendiculaire à l'axe longitudinal). Ce support est pourvu de moyens de manoeuvre et de verrouillage aptes à exercer sur ces moyens de support et d'accrochage une contrainte tendant à les rapprocher, entraînant un repli de la lentille souple sur elle-même et son maintien dans cette forme repliée.

Suivant un mode de réalisation préféré, le support est solidaire d'un bouchon d'un flacon hermétique apte à contenir un fluide stérile, tel que, par exemple, un sérum physiologique ou un gaz inerte.

Les moyens de verrouillage comprennent une pièce mobile coulissant parallèlement à l'axe longitudinal des membres flexibles.

La contrainte tendant à rapprocher les deux membres est produite de préférence par un effet de came entre la pièce mobile et les faces distales des membres, et avantageusement, cette contrainte tendant à rapprocher les deux membres est produite par un déplacement de la pièce mobile vers l'extrémité proximale de la tige.

Suivant un mode de réalisation préféré, ce déplacement de la pièce mobile dégage les moyens de support de la lentille de façon à faciliter le prélèvement de la lentille repliée sur elle-même.

Suivant un mode de réalisation avantageux, les moyens de verrouillage comprennent des crochets escamotables prévenant un déplacement axial de la lentille souple.

Suivant un mode de réalisation avantageux, les crochets comprennent des charnières actionnées par le déplacement des moyens de verrouillage.

D'autres particularités et avantages de l'invention ressortiront dans la description ci-après d'un mode de réalisation avantageux, référence étant faite aux dessins annexés dans lesquels :
la Fig. 1 est une vue éclatée, en perspective, de la partie centrale du support;
la Fig. 2 est une vue en perspective du dispositif de verrouillage;
la Fig. 3 est une vue en perspective du support assemblé;
les Fig. 4 et 5 sont des vues latérales schématiques du dispositif dans son ensemble, respectivement avant et après actionnement.

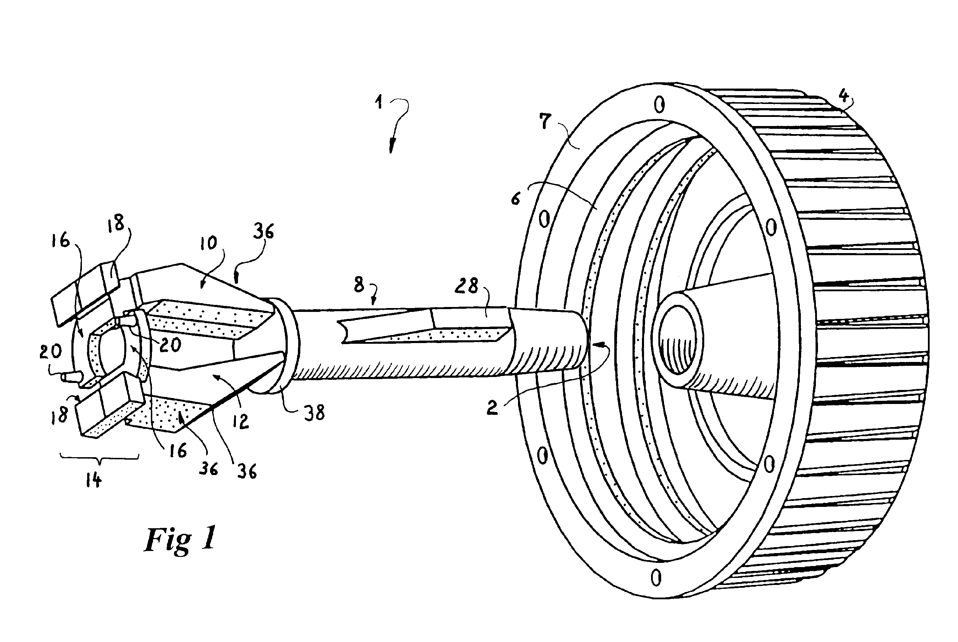

La Fig. 1 montre la partie centrale du dispositif 1 dans son ensemble, qui peut être, comme représenté ici, rendue solidaire par son extrémité proximale 2 d'un bouchon 4 d'un récipient (non représenté). Le bouchon 4 est doté d'un filet 6 et sert ici à la fois d'organe de manipulation pour le support et d'organe de scellement pour un récipient qui peut contenir un fluide assurant à la fois la stérilité et la bonne conservation d'une IOL.

On notera que le filet 6, suivant la forme du récipient, peut être mâle ou, comme représenté ici, femelle. Le bouchon 4 porte ici, en outre, une rondelle d'étanchéité 7.

La partie médiane du support 1 affecte la forme d'une tige 8. A l'extrémité distale de cette tige 8 s'articulent deux membres 10, 12 formant pince. L'extrémité libre de chacun de ces membres 10, 12 est munie d'un support de lentille 14. Ces supports de lentille 14 sont conçus pour un soutien et un maintien efficace d'une IOL même après une longue durée de stockage. La lentille est ici supportée perpendiculairement à l'axe de la tige 8. Chacun des supports 14 comprend des moyens de maintien, composés ici d'un segment de maintien 16, sensiblement perpendiculaire à l'axe de la tige 8, et d'une mâchoire 18 relié par un arrondi avec le segment 16, avec lequel elle fait un angle aigu. Chaque segment de maintien 16 est muni d'un ergot 20. Les membres 10, 12 pouvant se rapprocher angulairement l'un de l'autre, permettent d'exercer une contrainte sur une lentille souple placée entre leurs extrémités.

Lors du conditionnement, une IOL est montée sur un support 1 muni des moyens de maintien 16,18,20 correspondant à sa forme propre. Une très faible contrainte maintient la lentille plaquée contre les segments 16, empêchant tout déplacement axial. De surcroît, les mâchoires 18 et les ergots 20 préviennent tout déplacement latéral. Il va de soi que la forme et les dimensions des moyens de maintien 14, et notamment la présence éventuelle d'ergots 20, leur écartement et leur diamètre, sont déterminés par les dimensions nominales de chaque IOL.

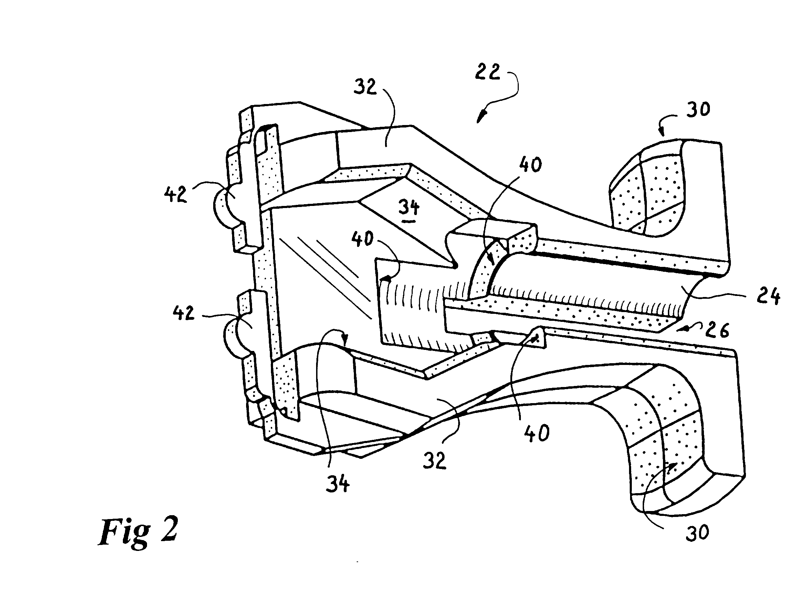

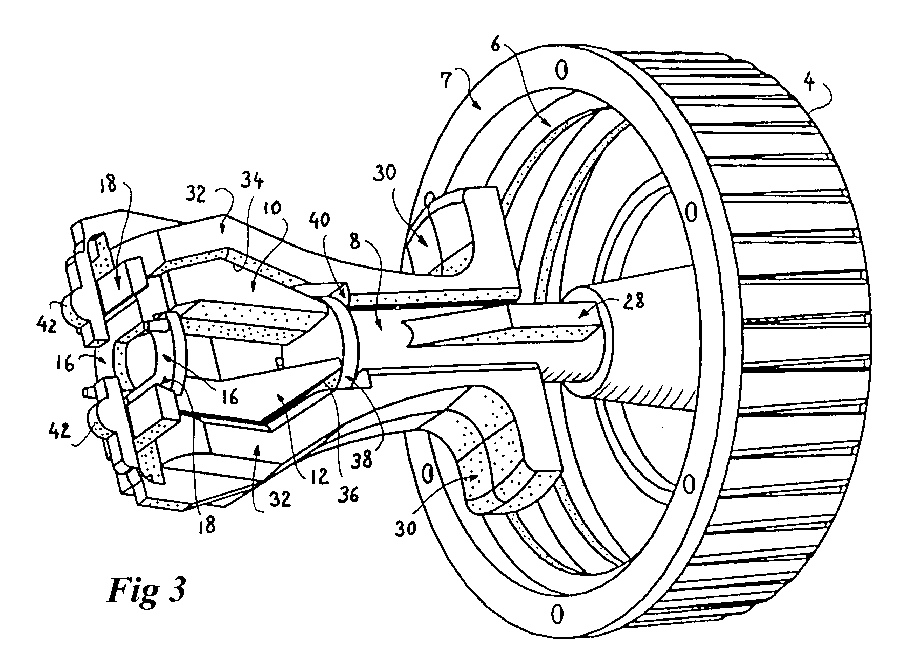

La Fig.2 représente une pièce mobile 22 servant à manoeuvrer et verrouiller le dispositif de l'invention. La pièce mobile 22, qui est généralement moulée, est, comme on peut le voir à la Fig. 3, encliquetable sur la tige 8. Elle comprend un coulisseau 24 et une saignée 26 coopérant avec une clavette longitudinale 28 incorporée à la tige 8 de façon à assurer un calage angulaire rigoureux de ces deux pièces lors de leur déplacement axial relatif.

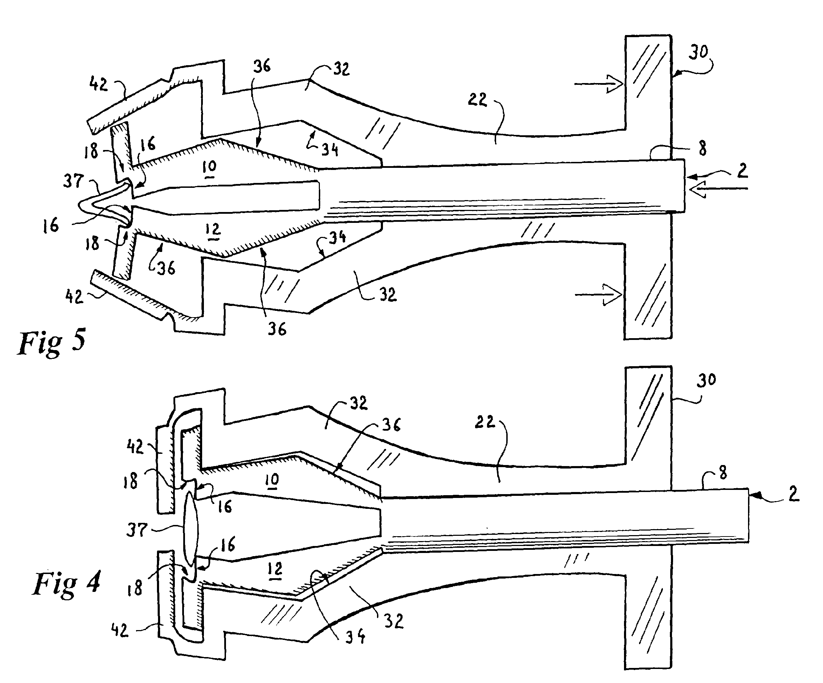

La partie proximale de la pièce mobile 22 s'évase de façon à former une double gâchette 30 permettant d'actionner le dispositif. Deux bras rigides 32 s'étendent à la partie distale de la pièce mobile, embrassant longitudinalement les membres 10,12. Les faces internes 34 de ces membres forment chacune un chemin de came 34. Comme on peut le voir en se référant à la Fig. 3, où le support est montré tout assemblé, et à la figure 5, ces faces coopèrent avec les faces distales 36 membres 10, 12, forçant les supports 14 à se rapprocher lorsque la pièce mobile 22 est déplacée vers l'extrémité proximale de la tige 8.

La Fig. 4 montre l'aspect du dispositif lorsque la pièce mobile 22 est écartée de la base de la tige 8. A ce moment, les moyens de maintien 14 exercent une contrainte juste suffisante pour y maintenir de façon sûre une IOL 37 en place.

Des moyens de butée 38, 40 empêchent la pièce mobile 22 de s'écarter de cette position durant le stockage.

Pour prévenir tout risque de séparation de l'IOL 37 de son support (notamment en cas de choc violent lors du transport), deux griffes 42 sont disposées parallèlement au plan de maintien de la lentille.

La Fig. 5 montre l'aspect que présente le support de l'invention lorsque l'opérateur a rapproché la pièce mobile 22 de la base de la tige 8 en pressant sur les gâchettes 30. La pièce mobile 22 s'étant dégagée de ses butées, les chemins de came 34 forcent les membres 10, 12 et donc les moyens de maintien 14 à se rapprocher, entraînant le pliage de l'IOL 37. En outre, le déplacement relatif des extrémités des membres 10, 12 force les griffes 42 (dont le rôle est terminé) à s'écarter. On remarquera que le sens de déplacement choisi dégage complètement l'IOL 37, rendant très aisé son prélèvement sous une grande variété d'angles. A ce stade, il n'est plus nécessaire que l'opérateur maintienne sa pression sur les gâchettes 30 : la pièce mobile 22 étant dans cette position, les mâchoires restent verrouillées en position fermée. Il est donc possible à l'opérateur, s'il le souhaite, de replacer à ce moment le support de l'IOL 37 dans son récipient et de procéder à d'autres préparatifs ou actions. Il lui est même possible de procéder à la présente opération dans une phase préparatoire de son intervention, ce qui permet un gain de temps et de sécurité, ou de déléguer ce soin à un de ses assistants.

Un avantage du dispositif est qu'il a un effet démultiplicateur : les manipulations centimétriques effectuées par l'opérateur (le déplacement des moyens de verrouillages) se traduisent par des déplacements millimétriques des supports, ce qui permet des mouvements plus naturels et diminue également le risque d'erreurs de manipulations.

On notera que dans l'exemple de réalisation présenté plus haut, les membres sont disposés de façon symétrique par rapport à un plan transversal. Il va de soi, cependant, que l'invention ne se limite pas à un tel mode de réalisation et que, par exemple, un des membres peut être de dimensions différentes ou même avoir une partie angulaire fixe par rapport au support.

La lentille peut également être maintenue dans un plan non pas perpendiculaire mais sensiblement parallèle à l'axe de déplacement des moyens de verrouillage, les moyens de support effectuant dans ce cas un déplacement relatif linéaire plutôt qu'angulaire les uns par rapport aux autres.

## Revendications

1. Support (1) de lentille intraoculaire pliable comprenant une tige (8) supportant deux membres flexibles (10, 12) formant pince s'étendant parallèlement à un axe longitudinal, caractérisé en ce que ces membres flexibles (10, 12) portent à leur extrémité distale, tournés l'un vers l'autre, des moyens de support et d'accrochage (14) aptes à maintenir une lentille intraoculaire souple (37) sensiblement dans un plan, ledit support (1) étant pourvu de moyens de manoeuvre et de verrouillage (22) aptes à exercer sur ces moyens de support et d'accrochage (14) une contrainte tendant à les rapprocher, entraînant un repli de la lentille souple (37) sur elle-même et son maintien dans cette forme repliée.

2. Support (1) suivant la revendication 1 caractérisé en ce que le plan de maintien de la lentille (37) est sensiblement perpendiculaire à l'axe longitudinal.

3. Support suivant l'une quelconque des revendications 1 et 2 caractérisé en ce que qu'il est solidaire d'un bouchon (4) d'un flacon hermétique apte à contenir un fluide stérile.

4. Support suivant l'une quelconque des revendications 1 à 3 caractérisé en ce que les moyens de verrouillage comprennent une pièce mobile (22) coulissant parallèlement à l'axe longitudinal des membres flexibles (10, 12).

5. Support suivant la revendication 4 caractérisé en ce que la contrainte tendant à rapprocher les deux membres (10, 12) est produite par un effet de came entre la pièce mobile (22) et les faces distales (36) des membres (10, 12).

6. Support suivant la revendication 5 caractérisé en ce que la contrainte tendant à rapprocher les deux membres est produite par un déplacement de la pièce mobile (22) vers l'extrémité proximale de la tige (8).

7. Support suivant la revendication 6 caractérisé en ce que le déplacement de la pièce mobile (22) dégage les moyens de support (14) de la lentille (37) de façon à faciliter le prélèvement de la lentille repliée sur elle-même.

8. Support suivant l'une quelconque des revendications précédentes, caractérisé en ce que les moyens de verrouillage (22) comprennent des crochets escamotables (42) aptes à prévenir un déplacement axial de la lentille souple (37).

9. Support suivant la revendication 8 caractérisé en ce que les crochets escamotables (42) comprennent des charnières actionnées par le déplacement des moyens de verrouillage (22).
